# EUROPEAN PATENT SPECIFICATION

(11) **EP 3 769 678 B1**
(45) Date of publication and mention of the grant of the patent: **06.05.2026**
(21) Application number: 19771643.4
(22) Date of filing: 21.03.2019
(51) Int. Cl.: A61B 5/1455, H01R 13/64

(54) **MULTI-COMPATIBILITY PULSE BLOOD OXYGEN SENSOR**
MULTIKOMPATIBLER PULSBLUTSAUERSTOFFSENSOR
CAPTEUR D'OXYMÉTRIE DE POULS MULTI-COMPATIBILITÉ

(30) Priority: 21.03.2018 CN 201810235474
(43) Date of publication of application: 27.01.2021
(73) Proprietor: Solaris Medical Technology, Inc., Shenzhen, Guangdong 518000 (CN)
(72) Inventor: WANG, Ganbing, Shenzhen, Guangdong 518000 (CN); XU, Chunhui, Shenzhen, Guangdong 518000 (CN)
(74) Representative: Vitina, Maruta
(86) International application number: PCT/CN2019/078947
(87) International publication number: WO 2019/179479

(56) References cited:
- WO-A1-2011/086520
- CN-A- 101 080 192
- CN-A- 102 670 211
- CN-A- 103 271 745
- CN-A- 104 853 672
- CN-A- 108 283 498
- CN-U- 208 591 050
- DE-U1- 202010 015 884
- US-A1- 2010 081 945
- US-A1- 2012 130 204

## Description

### TECHNICAL FIELD

The present application relates to pulse oximetry sensors, and more particularly to a pulse oximetry sensor compatible with multiple monitors.

### BACKGROUND

The monitor manufacturers vary in the oximetry technique, which renders their corresponding pulse oximetry sensors technically different from each other, so that a sensor produced by one manufacturer generally cannot be compatible with a monitor produced by another manufacturer.

However, actually, the monitors used in different departments are often produced by different manufacturers. For example, there may be four kinds of monitors used in the four clinical environments that a patient may undergo in one treatment course, that is, monitor A is used in the operating room; monitor B is used in the postanesthesia care unit; monitor C is used in the intensive care unit (ICU); and monitor D is used in the general ward, which indicates that four kinds of pulse oximetry sensors are required to be prepared to satisfy the clinical requirements during the same treatment course.

Considering the above, since different monitors generally have different ports, it is necessary to use four kinds of pulse oximetry sensors respectively compatible with the four different monitors in the same treatment course, which makes for an increase not only in the clinical workload and operation complexity, but also in the treatment cost.

Publication WO2011086520A1 discloses a multi-system compatible pulse oximetry sensor including a light emitting unit 3. There are three LEDs being connected in parallel in the initial LED circuit. However, there are only three leads D circuit and LED circuit variation can create only two_configurations.

### SUMMARY

An object of this application is to provide a pulse oximetry sensor compatible with various monitors, simplifying the operation and lowering the cost.

The invention is as defined in the appended set of claims.

Hereinafter, any contents that do not correspond to the scope of the claims are only for illustrative purpose, or to highlight the effect of a particular aspect or feature.

Compared to the prior art, there are the following beneficial effects.

A combination of the multi-compatible pulse oximetry sensor and an adapter or an adapter cable is employed, and at least three light emitting diodes including red light emitting diodes and infrared light emitting diodes are connected in series at the detection end of the pulse oximetry sensor. Moreover, the adapter or the adapter cable can be customized according to the target monitor. Therefore, the pulse oximetry sensor can be compatible with multiple monitors, simplifying the operation and lowering the cost.

### BRIEF DESCRIPTION OF THE DRAWINGS

Fig. 1 is a block diagram schematically depicting a multi-compatible pulse oximetry sensor.
Fig. 2 is a block diagram schematically depicting a multi-compatible pulse oximetry sensor combination.
Fig. 3 schematically illustrates a circuit of a light emitter in the multi-compatible pulse oximetry sensor.
Fig. 4 is a front view of the circuit shown in Fig. 3 after packaged.
Fig. 5 is a rear view of the circuit shown in Fig. 3 after packaged.
Fig. 6 schematically illustrates a structure of the multi-compatible pulse oximetry sensor.
Fig. 7 schematically illustrates a structure of an adapter cable.
Fig. 8 schematically illustrates another structure of an adapter cable.
Fig. 9 schematically illustrates another structure of an adapter cable.
Fig. 10 is a block diagram schematically depicting another multi-compatible pulse oximetry sensor combination.
Fig. 11 schematically shows a structure of an adapter.

In the drawings, 10-patient; 30-monitor; 50-multi-compatible pulse oximetry sensor; 501-detection end; 5015-light emitter; 503-output end; 505-cable; 70, 70a, 70b, 70c-adapter cable; 701-first end for connecting the sensor; 703-second end for connecting the monitor; 705, 705a, 705b, 705c-adapter wire; 80-adapter cable; 90-adapter; 901-first adapter end; 903-second adapter end; 905-connector; and 100, 100a-multi-compatible pulse oximetry sensor combination.

### DETAILED DESCRIPTION OF EMBODIMENTS

The invention will be illustrated below in detail with reference to the accompanying drawings.

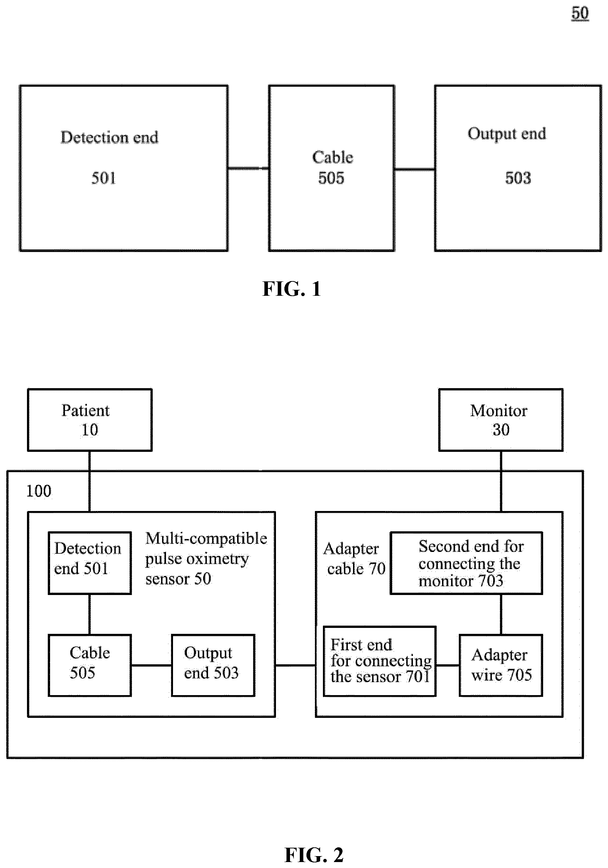

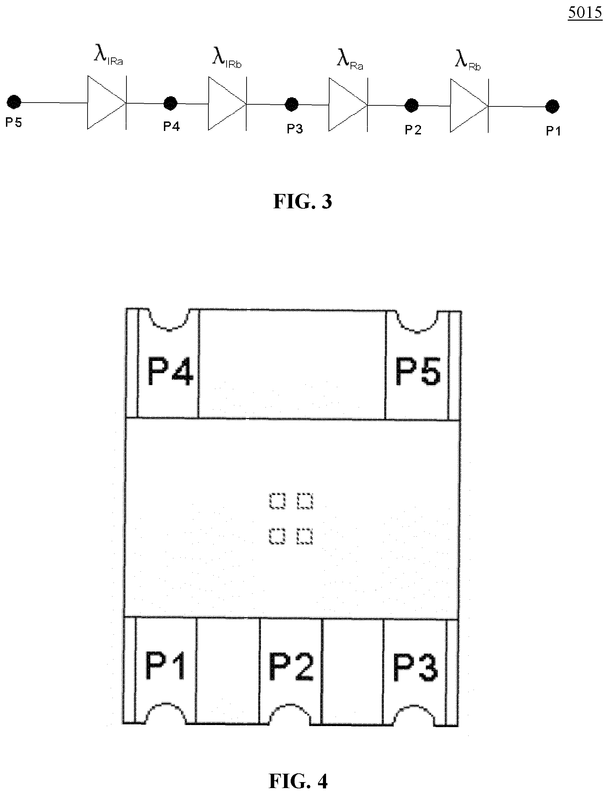

Fig. 1 is a block diagram schematically depicting a multi-compatible pulse oximetry sensor. Fig. 2 is a block diagram schematically depicting a multi-compatible pulse oximetry sensor combination. Fig. 3 schematically illustrates a circuit of a light emitter in the multi-compatible pulse oximetry sensor. In an embodiment, this application provides a multi-compatible pulse oximetry sensor combination 100, which is connected to a monitor 30 to obtain the blood oxygen saturation of a patient 10 by pulse oximetry. The multi-compatible pulse oximetry sensor combination 100 is composed of a multi-compatible pulse oximetry sensor 50 and an adapter cable 70 connected to the multi-compatible pulse oximetry sensor 50.

The multi-compatible pulse oximetry sensor 50 of the invention is compatible with multiple types of monitors 30, and includes: a detection end 501, an output end 503 and a cable 505 for connecting the detection end 501 with the output end 503. The detection end 501 is provided with a light emitter 5015 and a photodetector (not shown in the drawings). The light emitter 5015 is composed of at least three light emitting diodes, and the at least three light emitting diodes are connected in series. In an embodiment, the at least three light emitting diodes consist of at least two red light emitting diodes varying in peak wavelengths and at least one infrared light emitting diode. In an embodiment, the at least three light emitting diodes consist of at least one red light emitting diode and at least two infrared light emitting diodes varying in peak wavelengths. The detection end 501 is connected to an output port of the output end 503 through a conductive wire in the cable 505.

This application also provides a method of operating the multi-compatible pulse oximetry sensor 50 of the invention, which is specifically described as follows. The multi-compatible pulse oximetry sensor 50 and an adapter cable 70 adapting to the multi-compatible pulse oximetry sensor 50 are prepared, where the adapter cable 70 includes a first end 701 for connecting the multi-compatible pulse oximetry sensor 50, a second end 703 for connecting the monitor 30 and an adapter wire 705 for connecting the first end 701 and the second end 703 of the adapter cable 70. The adapter cable 70 can be customized for a specific monitor, so as to selectively electrically connect specific red light emitting diode and infrared light emitting diode matching the monitor. Finally, the multi-compatible pulse oximetry sensor 50 is placed on the patient 10, and the multi-compatible pulse oximetry sensor 50 is connected with a connector of the monitor 30 through the adapter cable 70 to collect the blood oxygen information of the patient 10.

The combination of the light emitting diodes in the light emitter 5015 can be adjusted according to the peak wavelength which can be recognized by the monitor 30. The light emitting diodes with overlapping peak wavelength are combined. The multi-compatible pulse oximetry sensor 50 of the invention can be applied to at least two types of monitors at the same time, and can ensure the measurement accuracy to meet the clinical requirement. The invention employs the adapter cable to selectively electrically connect the red light emitting diodes and the infrared light emitting diodes corresponding to the monitor 30 to achieve the adaptation of the multi-compatible pulse oximetry sensor 50 to the monitor 30, ensuring the measurement accuracy.

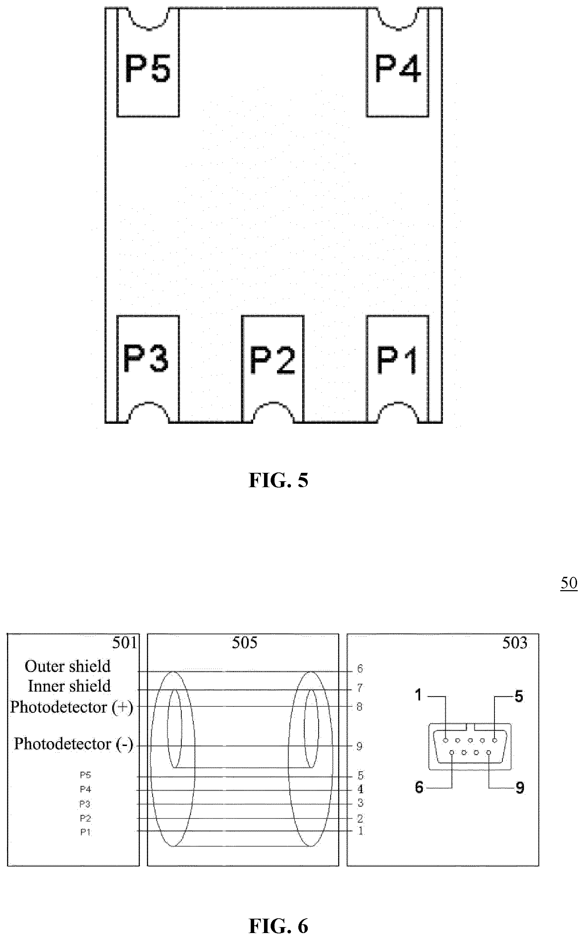

Fig. 3 schematically illustrates a circuit of the light emitter in the multi-compatible pulse oximetry sensor according to an embodiment of the invention. Fig. 4 is a front view of the circuit shown in Fig. 3 after packaged. Fig. 5 is a rear view of the circuit shown in Fig. 3 after packaged. Fig. 6 schematically illustrates a structure of the multi-compatible pulse oximetry sensor according to an embodiment of the invention. In an embodiment, the peak wavelength of each red light emitting diode and each infrared light emitting diodes can be determined according to the calibration curve of the monitor.

For example, in this case that a light combination recognized by monitor A is λ*_{Ra}* (peak wavelength of red light) and λ*_{IRa}* (peak wavelength of infrared light), a light combination recognized by monitor B is λ*_{Rb}* and λ*_{IRb}*, and a light combination recognized by monitor C is λ*_{Ra}* and λ*_{IRb}*, a light emitter 5015 consisting of two red light emitting diodes and two infrared light emitting diodes is selected to enable the multi-compatible pulse oximetry sensor 50 to be compatible with the above three types of monitors 30, where the peak wavelengths of the two red light emitting diodes are λ*_{Ra}* and λ*_{Rb}*, respectively, and the peak wavelengths of the two infrared light emitting diodes are λ*_{IRa}* and λ*_{IRB}*, respectively. The two red light emitting diodes are connected in series to form the first series circuit, and the two infrared light emitting diodes are connected in series to form the second series circuit. The four light emitting diodes each are a light emitting diode (LED). The first series circuit is connected with the second series circuit in series to form five connection ports (P1, P2, P3, P4 and P5). The five connection ports are connected with five output ports at the output end 503 through five wires in the cable 505, respectively. For a given monitor, it is only required to connect the corresponding connection port to allow the corresponding light emitting diode to emit the light of specific peak wavelength which can be recognized by the given monitor. In the actual application, it is feasible to accordingly select a peak wavelength according to the monitor 30 required to be used, which can ensure the measurement error of the multi-compatible pulse oximetry sensor 50 to be within ±2%.

In an embodiment, in order to enable the multi-compatible pulse oximetry sensor 50 to be compatible with the above three types of monitors 30, a light emitter 5015 composed of one red light emitting diode and two infrared light emitting diodes is used, where the peak wavelength of the red light emitting diode is a median value in a range from the upper limit of λ*_{Ra}* to the lower limit of λ*_{Rb}*, and the upper limit of *Ra* is greater than the lower limit of *Rb.* The peak wavelengths of the two infrared light emitting diodes are λ*_{IRa}* and λ*_{IRb}*, respectively, and thus it is possible to adopt merely one red light emitting diode in the practical application. These light emitting diodes each are an LED. The two infrared light emitting diodes are connected in series to form the series circuit, which is further connected with the one red light emitting diode in series to form four connection ports in the light emitter 5015. The four connection ports are connected to the four output ports of the output end 503 through four wires in the cable 505, respectively. Compared to the method mentioned above, this method has a relatively larger measurement error (within ±3%) in the practical application, but it can still meet the clinical requirements. Moreover, this method can significantly lower the production cost of the multi-compatible pulse oximetry sensor 50.

In an embodiment, the light emitter 5015 is composed of two red light emitting diodes and one infrared light emitting diode, where the two red light emitting diodes are connected in series, and the resulting series circuit is then connected with the infrared light emitting diode in series to form four connection ports in the light emitter 5015. The four connection ports are connected to the four output ports of the output end 503 through four wires in the cable 505, respectively.

In an embodiment, the four light emitting diodes are integrally packaged, that is, the four light emitting diodes (such as LEDs) are packaged in one chip (the light emitter 5015 is composed of a single chip). In an embodiment, the light emitting diodes can be expanded to be the same as the ports at the output end 503 in number. The more the peak wavelengths the light emitting diodes have, the more the types of monitors the multi-compatible pulse oximetry sensor 50 can be compatible with at the same time.

In an embodiment, in order to increase the flexibility in the peak wavelength selection and configuration and improve the measurement accuracy of the multi-compatible pulse oximetry sensor 50, the red or infrared light emitting diodes can be individually packaged in one small emitting diode unit (single chip). The emitting diode units with multiple peak wavelengths can be prepared in advance, so that in the manufacturing process of the multi-compatible pulse oximetry sensor 50, it is possible to select the emitting diode units with specific peak wavelengths according to the actual needs, and assemble these emitting diode units by mounting and welding to form an integrated circuit (i.e., chip) as the light emitter 5015 of the multi-compatible pulse oximetry sensor 50 (that is, the light emitter 5015 is composed of multiple chips). Therefore, in order to expand the adaptation range of the oximetry sensor, it is only required to prepare multiple emitting diode units of different peak wavelengths in advance.

The four emitting diode units included in the above light emitter 5015 are packaged individually. In an embodiment, the light emitting diodes can also be packaged in the form of combination. For example, in the case that there are five light emitting diodes in the light emitter 5015, three of the five light emitting diodes are packaged into one emitting diode unit and the other two light emitting diodes are packaged into one emitting diode unit. The five light emitting diodes can also be packaged in other combinations.

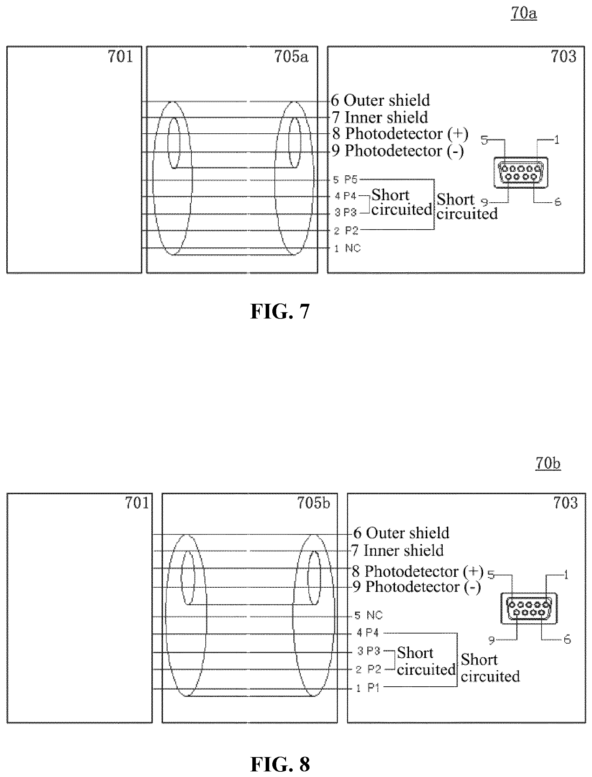

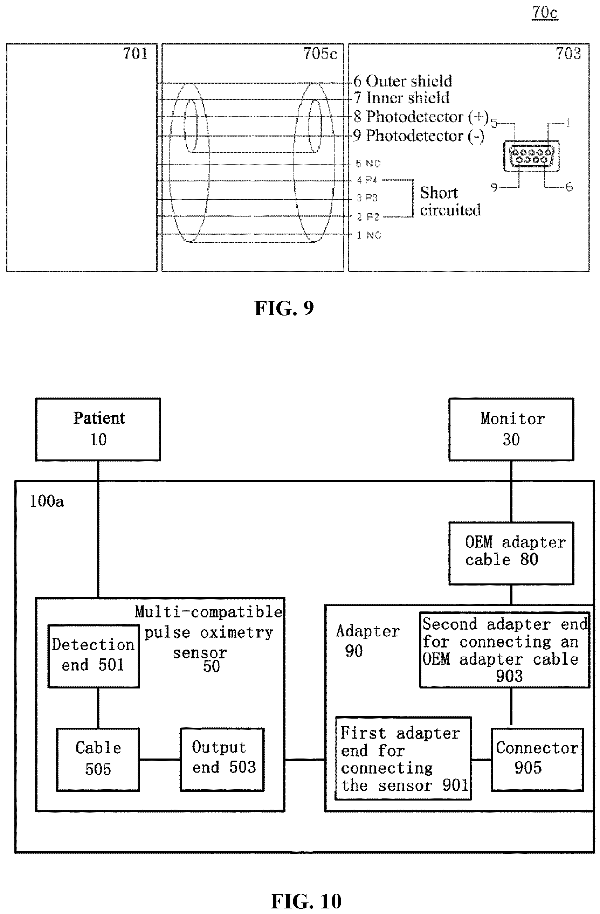

Fig. 7 schematically illustrates another structure of an adapter cable 70a. Fig. 8 schematically illustrates another structure of an adapter cable 70b. Fig. 9 schematically illustrates another structure of an adapter cable 70c. The adapter cable 70a, 70b or 70c includes a first end 701 for connecting the multi-compatible pulse oximetry sensor 50, a second end 703 for connecting the monitor 30 and adapter wires 705a, 705b or 705c for connecting the first end 701 with the second end 703. The adapter cable 70a, 70b or 70c can be customized according to the monitor 30 to connect the first end 701 with the second end 703. The first end 701 can adapt to the output end 503 of the multi-compatible pulse oximetry sensor 50 to selectively electrically connect the red and infrared light emitting diodes corresponding to the specific monitor 30.

In an embodiment, in order to adapt to the monitor A, the first adapter wires 705a in the first adapter cable 70a are configured to short-circuit the port 2 (corresponding to the connection port 2 of the detection end 501) with the port 5 (corresponding to the connection port 5 of the detection end 501) of the second end (703), and short-circuit the port 3 (corresponding to the connection port 3 of the detection end 501) with the port 4 (corresponding to the connection port 4 of the detection end 501) of the second end 703. The port 1 (corresponding to the connection port 1 of the detection end 501) of the second end 703 is not used (namely, NC). It can be understood that the first adapter cable 70a specifically electrically connect the red light emitting diode with a peak wavelength of λ*_{Ra}*and the infrared light emitting diode with a peak wavelength of λ*_{IRa}* to enable the multi-compatible pulse oximetry sensor 50 to be compatible with the monitor A.

In an embodiment, in order to adapt to the monitor B, the second adapter wires 705b in the second adapter cable 70b are configured to short-circuit the port 2 (corresponding to the connection port 2 of the detection end 501) with the port 3 (corresponding to the connection port 3 of the detection end 501) of the second end 703, and short-circuit the port 1 (corresponding to the connection port 1 of the detection end 501) with the port 4 (corresponding to the connection port 4 of the detection end 501) of the second end 703. The port 5 (corresponding to the connection port 5 of the detection end 501) of the second end 703 is not used (namely, NC). It can be understood that the second adapter cable 70b specifically electrically connect the red light emitting diode with a peak wavelength of λ*_{Rb}*and the infrared light emitting diode with a peak wavelength of λ*_{IRb}* to enable the multi-compatible pulse oximetry sensor 50 to be compatible with the monitor B.

In an embodiment, in order to adapt to the monitor C, the third adapter wires 705c in the third adapter cable 70c are configured to short-circuit the port 2 (corresponding to the connection port 2 of the detection end 501) with the port 4 (corresponding to the connection port 4 of the detection end 501) of the second end 703. The port 1 (corresponding to the connection port 1 of the detection end 501) and the port 5 (corresponding to the connection port 1 of the detection end 501) of the second end 703 are not used (namely, NC). It can be understood that the third adapter cable 70c specifically electrically connect the red light emitting diode with a peak wavelength of λ*_{Ra}*and the infrared light emitting diode with a peak wavelength of λ*_{IRb}* to enable the multi-compatible pulse oximetry sensor 50 to be compatible with the monitor C.

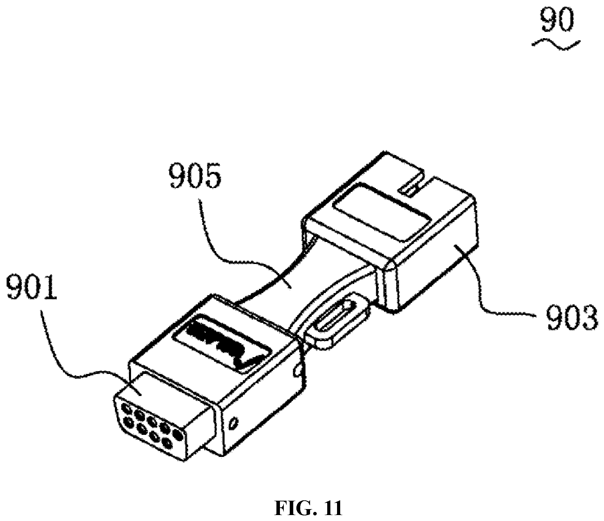

Fig. 10 is a block diagram schematically depicting another multi-compatible pulse oximetry sensor combination. Fig. 11 schematically shows a structure of an adapter according to an embodiment of the invention. This application provides another multi-compatible pulse oximetry sensor combination 100a, which is connected to the monitor 30 for measuring the pulse blood oxygen saturation of the patient 10. The multi-compatible pulse oximetry sensor combination 100a is composed of a multi-compatible pulse oximetry sensor 50, an adapter 90 connected to the multi-compatible pulse oximetry sensor 50 and an adapter cable 80 connected to the adapter 90.

The adapter 90 of the invention can be customized according to a specific monitor 30, and includes a first adapter end 901, a second adapter end 903 and a connector 905 for connecting the first adapter end 901 with the second adapter end 903. The first adapter end 901 adapts to the output end 503 of the multi-compatible pulse oximetry sensor 50, and the second adapter end 903 adapts to the adapter cable 80, so as to selectively electrically connect the specific red light emitting diode and infrared light emitting diode matching the monitor 30. The adapter cable 80 refers to an adapter cablethat is compatible with the monitor 30. The adapter 90 is connected in series between the multi-compatible pulse oximetry sensor 50and the adapter cable 80.

This application also provides another method of operating the multi-compatible pulse oximetry sensor 50, which is specifically described as follows. The multi-compatible pulse oximetry sensor 50 and an adapter 90 are prepared. The multi-compatible pulse oximetry sensor 50 is placed on the patient 10, and the adapter (90) is connected between the multi-compatible pulse oximetry sensor 50 and the adapter cable 80 connected to the specific monitor 30, so as to collect the blood oxygen information of the patient 10.

As described above, the multi-compatible pulse oximetry sensor 50 of the invention can be connected to the monitor 30 through the adapter cable 70 (i.e., the first multi-compatible pulse oximetry sensor combination 100), and can also be connected to the connector on the monitor 30 through the adapter 90 and the adapter cable 80 connected to the adapter 90 (i.e., the second multi-compatible pulse oximetry sensor combination 100a).

Compared to the prior art, there are the following beneficial effects.

A combination of the multi-compatible pulse oximetry sensor 50 and an adapter 90 or an adapter cable 70 is employed, and at least three light emitting diodes including red light emitting diodes and infrared light emitting diodes are connected in series at the detection end 501 of the multi-compatible pulse oximetry sensor 50. Moreover, the adapter 90 or the adapter cable 70 can be customized according to the target monitor 30. Therefore, the pulse oximetry sensor can be compatible with multiple monitors, simplifying the operation and lowering the cost.

## Claims

1. A multi-compatible pulse oximetry sensor (50), comprising:
a detection end (501);
an output end (503); and
a cable (505) configured to connect the detection end with the output end;
wherein the detection end is provided with a light emitter and a photodetector; the light emitter is composed of a plurality of light emitting diodes and a plurality of connection ports; the light emitting diodes consist of at least two red light emitting diodes varying in peak wavelengths and at least one infrared light emitting diode or consist of at least one red light emitting diode and at least two infrared light emitting diodes varying in peak wavelengths; and each connection port is connected to an output port of the output end through a conductive wire in the cable;
**characterized in that** the light emitting diodes are connected in series at the detection end;
wherein the light emitter is composed of two red light emitting diodes and two infrared light emitting diodes; the two red light emitting diodes are connected in series to form a first series circuit; the two infrared light emitting diodes are connected in series to form a second series circuit; the first series circuit is connected with the second series circuit in series; the light emitter is provided with five connection ports; the cable has five conductive wires; the output end has five output ports; and the five connection ports of the light emitter are electrically connected with the five output ports through the five conductive wires, respectively; or
wherein the light emitter is composed of one red light emitting diode and two infrared light emitting diodes; the two infrared light emitting diodes are connected in series to form a series circuit; the one red light emitting diode is connected in series with the series circuit; the light emitter is provided with four connection ports; the cable has four conductive wires; the output end has four output ports; and the four connection ports of the light emitter are electrically connected with the four output ports through the four conductive wires, respectively; or
wherein the light emitter is composed of two red light emitting diodes and one infrared light emitting diode; the two red light emitting diodes are connected in series to form a series circuit; the one infrared light emitting diode is connected in series with the series circuit; the light emitter is provided with four connection ports; the cable has four conductive wires; the output end has four output ports; and the four connection ports of the light emitter are electrically connected with the four output ports through the four conductive wires, respectively.

2. The multi-compatible pulse oximetry sensor according to claim 1, **characterized in that** the light emitting diodes are packaged together in one chip, and the chip is provided at the detection end; or
the light emitting diodes are individually packaged in a plurality of chips, and the chips are all provided at the detection end.

3. A multi-compatible pulse oximetry sensor combination (100), comprising:
the multi-compatible pulse oximetry sensor according to any one of claims 1-2; and an adapter cable (70)
for connecting the multi-compatible pulse oximetry sensor with a monitor, wherein the adapter cable comprises a first adapter end configured to connect the multi-compatible pulse oximetry sensor; a second adapter end configured to connect the monitor; and an adapter wire for connecting the first adapter end with the second adapter end.

4. A multi-compatible pulse oximetry sensor combination (100a), comprising:
the multi-compatible pulse oximetry sensor according to any one of claims 1-2;
an adapter cable; and
an adapter (90) for electrically connecting the multi-compatible pulse oximetry sensor with the adapter cable;
wherein the adapter comprises a first adapter end configured to connect the multi-compatible pulse oximetry sensor; a second adapter end configured to connect the adapter cable; and a connector for connecting the first adapter end with the second adapter end.

## Patentansprüche

1. Multikompatibler Pulsoximetriesensor (50), umfassend:
ein Detektionsende (501);
ein Ausgangsende (503); und
ein Kabel (505), das dazu konfiguriert ist, das Detektionsende mit dem Ausgangsende zu verbinden;
wobei das Detektionsende mit einem Lichtemitter und einem Fotodetektor ausgestattet ist; der Lichtemitter aus einer Vielzahl von Leuchtdioden und einer Vielzahl von Anschlussports aufgebaut ist; die Leuchtdioden entweder aus mindestens zwei roten Leuchtdioden mit variierenden Spitzenwellenlängen und mindestens einer Infrarot-Leuchtdiode oder aus mindestens einer roten Leuchtdiode und mindestens zwei Infrarot-Leuchtdioden mit variierenden Spitzenwellenlängen bestehen; und jeder Anschlussport über einen Leitungsdraht im Kabel mit einem Ausgangsport des Ausgangsendes verbunden ist;
**dadurch gekennzeichnet, dass** die Leuchtdioden am Detektionsende in Reihe geschaltet sind;
wobei der Lichtemitter aus zwei roten Leuchtdioden und zwei infraroten Leuchtdioden aufgebaut ist; die zwei roten Leuchtdioden in Reihe geschaltet sind, um eine erste Reihenschaltung zu bilden; die zwei infraroten Leuchtdioden in Reihe geschaltet sind, um eine zweite Reihenschaltung zu bilden; die erste Reihenschaltung mit der zweiten Reihenschaltung in Reihe geschaltet ist; der Lichtemitter mit fünf Anschlussports versehen ist; das Kabel fünf Leitungsdrähte aufweist; das Ausgangsende fünf Ausgangsports aufweist; und die fünf Anschlussports des Lichtemitters elektrisch jeweils über die fünf Leitungsdrähte mit den fünf Ausgangsports verbunden sind; oder
wobei der Lichtemitter aus einer roten Leuchtdiode und zwei infraroten Leuchtdioden aufgebaut ist; die zwei infraroten Leuchtdioden in Reihe geschaltet sind, um eine Reihenschaltung zu bilden; die eine rote Leuchtdiode mit der Reihenschaltung in Reihe geschaltet ist; der Lichtemitter mit vier Anschlussports versehen ist; das Kabel vier Leitungsdrähte aufweist; das Ausgangsende vier Ausgangsports aufweist; und die vier Anschlussports des Lichtemitters elektrisch jeweils über die vier Leitungsdrähte mit den vier Ausgangsports verbunden sind; oder
wobei der Lichtemitter aus zwei roten Leuchtdioden und einer infraroten Leuchtdiode aufgebaut ist; die zwei roten Leuchtdioden in Reihe geschaltet sind, um eine Reihenschaltung zu bilden; die eine infrarote Leuchtdiode mit der Reihenschaltung in Reihe geschaltet ist; der Lichtemitter mit vier Anschlussports versehen ist; das Kabel vier Leitungsdrähte aufweist; das Ausgangsende vier Ausgangsports aufweist; und die vier Anschlussports des Lichtemitters elektrisch jeweils über die vier Leitungsdrähte mit den vier Ausgangsports verbunden sind.

2. Multikompatibler Pulsoximetriesensor nach Anspruch 1, **dadurch gekennzeichnet, dass** die Leuchtdioden gemeinsam auf einem Chip verkapselt sind und der Chip am Detektionsende vorgesehen ist; oder
die Leuchtdioden einzeln in einer Vielzahl von Chips verkapselt sind und die Chips sämtlich am Detektionsende angeordnet sind.

3. Multikompatible Pulsoximetriesensoranordnung (100), umfassend:
den Multikompatiblen Pulsoximetriesensor nach einem der Ansprüche 1 bis 2; und
ein Adapterkabel (70) zum Verbinden des multikompatiblen Pulsoximetriesensors mit einem Monitor, wobei das Adapterkabel ein erstes Adapterende zum Verbinden mit dem mehrkompatiblen Pulsoximetriesensor, ein zweites Adapterende zum Verbinden mit dem Monitor und eine Adapterleitung zum Verbinden des ersten Adapterendes mit dem zweiten Adapterende umfasst.

4. Multikompatible Pulsoximetriesensoranordnung (100a), umfassend:
den multikompatiblen Pulsoximetriesensor nach einem der Ansprüche 1 bis 2;
ein Adapterkabel; und
einen Adapter (90) zum elektrischen Verbinden des Multikompatiblen Pulsoximetriesensors mit dem Adapterkabel;
wobei der Adapter ein erstes Adapterende zum Verbinden mit dem multikompatiblen Pulsoximetriesensor, ein zweites Adapterende zum Verbinden mit dem Adapterkabel und einen Stecker zum Verbinden des ersten Adapterendes mit dem zweiten Adapterende umfasst.

## Revendications

1. Capteur d'oxymétrie de pouls multicompatible (50), comprenant :
une extrémité de détection (501) ;
une extrémité de sortie (503) ; et
un câble (505) configuré pour relier l'extrémité de détection à l'extrémité de sortie ;
dans lequel l'extrémité de détection est pourvue d'un émetteur lumineux et d'un photodétecteur ; l'émetteur lumineux est constitué d'une pluralité de diodes électroluminescentes et d'une pluralité de ports de connexion ; les diodes électroluminescentes sont constituées d'au moins deux diodes électroluminescentes rouges ayant des longueurs d'onde de crête différentes et d'au moins une diode électroluminescente infrarouge, ou sont constituées d'au moins une diode électroluminescente rouge et d'au moins deux diodes électroluminescentes infrarouges ayant des longueurs d'onde de crête différentes ; et chaque port de connexion est relié à un port de sortie de l'extrémité de sortie par un fil conducteur dans le câble ;
**caractérisé en ce que** les diodes électroluminescentes sont connectées en série au niveau de l'extrémité de détection ;
dans lequel l'émetteur lumineux est constitué de deux diodes électroluminescentes rouges et de deux diodes électroluminescentes infrarouges ; les deux diodes électroluminescentes rouges sont connectées en série pour former un premier circuit en série ; les deux diodes électroluminescentes infrarouges sont connectées en série pour former un second circuit en série ; le premier circuit en série est connecté en série avec le second circuit en série ; l'émetteur lumineux est pourvu de cinq ports de connexion ; le câble comporte cinq fils conducteurs ; l'extrémité de sortie comporte cinq ports de sortie ; et les cinq ports de connexion de l'émetteur lumineux sont électriquement reliés respectivement aux cinq ports de sortie par les cinq fils conducteurs ; ou
dans lequel l'émetteur lumineux est constitué d'une diode électroluminescente rouge et de deux diodes électroluminescentes infrarouges ; les deux diodes électroluminescentes infrarouges sont connectées en série pour former un circuit en série ; la diode électroluminescente rouge est connectée en série avec le circuit en série ; l'émetteur lumineux est pourvu de quatre ports de connexion ; le câble comporte quatre fils conducteurs ; l'extrémité de sortie comporte quatre ports de sortie ; et les quatre ports de connexion de l'émetteur lumineux sont électriquement reliés respectivement aux quatre ports de sortie par les quatre fils conducteurs ; ou
dans lequel l'émetteur lumineux est constitué de deux diodes électroluminescentes rouges et d'une diode électroluminescente infrarouge ; les deux diodes électroluminescentes rouges sont connectées en série pour former un circuit en série ; la diode électroluminescente infrarouge est connectée en série avec le circuit en série ; l'émetteur lumineux est pourvu de quatre ports de connexion ; le câble comporte quatre fils conducteurs ; l'extrémité de sortie comporte quatre ports de sortie ; et les quatre ports de connexion de l'émetteur lumineux sont électriquement reliés respectivement aux quatre ports de sortie par les quatre fils conducteurs.

2. Capteur d'oxymétrie de pouls multicompatible selon la revendication 1, **caractérisé en ce que** les diodes électroluminescentes sont encapsulées ensemble dans une même puce, et la puce est disposée au niveau de l'extrémité de détection ; ou
les diodes électroluminescentes sont encapsulées individuellement dans une pluralité de puces, et lesdites puces sont toutes disposées au niveau de l'extrémité de détection.

3. Ensemble de capteur d'oxymétrie de pouls multicompatible (100), comprenant :
le capteur d'oxymétrie de pouls multicompatible selon l'une quelconque des revendications 1 à 2 ; et
un câble adaptateur (70) destiné à relier le capteur d'oxymétrie de pouls multicompatible à un moniteur, dans lequel le câble adaptateur comprend une première extrémité d'adaptation configurée pour être connectée au capteur d'oxymétrie de pouls multicompatible ; une seconde extrémité d'adaptation configurée pour être connectée au moniteur ; et un fil adaptateur reliant la première extrémité d'adaptation à la seconde extrémité d'adaptation.

4. Ensemble de capteur d'oxymétrie de pouls multicompatible (100a), comprenant :
le capteur d'oxymétrie de pouls multicompatible selon l'une quelconque des revendications 1 à 2 ;
un câble adaptateur ; et
un adaptateur (90) pour relier électriquement le capteur d'oxymétrie de pouls multicompatible au câble adaptateur ;
dans lequel l'adaptateur comprend une première extrémité d'adaptation configurée pour être connectée au capteur d'oxymétrie de pouls multicompatible ; une seconde extrémité d'adaptation configurée pour être connectée au câble adaptateur ; et un connecteur reliant la première extrémité d'adaptation à la seconde extrémité d'adaptation.
